# EUROPEAN PATENT APPLICATION

(11) **EP 2 590 153 A1**
(43) Date of publication of application: **08.05.2013**
(21) Application number: 12191016.0
(22) Date of filing: 02.11.2012
(51) Int. Cl.: G09F 3/00, A61F 5/00, A41F 9/02

(54) **A Weight Management Device**

(30) Priority: 02.11.2011 GB 201118893
(71) Applicant: Banderis, Julia Mary, London Greater London W4 3BJ (GB)
(72) Inventor: Banderis, Julia Mary, London Greater London W4 3BJ (GB)
(74) Representative: Hill, Richard

(57) **Abstract**

A weight management device for moderating bodyweight by providing an indication of changes in body weight. The device comprises a length of non-extensible, pliable material with a non-releasable fastening means such that when placed around the wearer's waist it will tighten in response to overeating and will prevent further readjustment of the device's fastening to accommodate further weight gain.

## Description

The present invention relates to a weight management device and more specifically relates to a weight management device that can be worn unobtrusively around the waist.

There are many types of belt-like devices currently available that wrap around the waist that aim to either reduce weight or at least to reduce the waist measurement of the wearer, by various means.

Such devices may comprise an elasticated band or belt worn around a wearer's waist being removably secured by at least one fastening means. Said band or belt is usually sufficiently elasticated to have a compressive effect around the waist, thus temporarily reducing the waist measurement of the wearer whilst the device is being worn. Such devices are usually worn temporarily and over a short period of time, to create the impression of a smaller waist.

Alternative embodiments of the afore-described elasticated band devices may further comprise microcurrent devices that provide an electrical stimulation to muscles lying substantially adjacent to the device, which is claimed to encourage weight loss. Again, such devices are intended to be worn on a short term basis.

Other examples of similar belt-like devices may comprise a non-elasticated band or belt that is again, located around a wearer's waist that further comprises an adjustable fastener. Such a weight management device may further comprise measurement graduations that identify the waist measurement of the wearer, such that they may monitor any fluctuations in waist measurement and thus monitor their weight based on these measurements.

Whilst devices such as those described above may provide a useful indication of fluctuations in waist measurements that may be used as a relative indication of weight loss or gain, such devices may not accurately assist the wearer in managing their weight from a dietary and/or lifestyle awareness perspective.

For example, devices comprising elasticated fabrics will obviously expand by virtue of their construction, meaning that there may be the potential for a wearer's waist measurement to significantly expand before the wearer becomes aware of a significant increase in weight, as the elasticated nature of the fabric will often compensate for slight fluctuations in waist circumference.

This means therefore, that by the time a wearer becomes aware of an increase in waist measurement, it is likely to be at the point whereby the elasticated fabric is approaching maximum distortion, thus causing the device to feel tighter around the wearer's waist, thus alerting them to potential weight gain. By this point, the wearer may have gained significant weight and therefore the process of regaining the original weight will arguably take longer and be more challenging.

Furthermore, the adjustable and releasable nature of many of the existing belt-like devices enables a wearer to easily circumvent the tension created by the band as a result of over-eating, by simply adjusting the band to increase the circumference. This effectively permits the wearer to disregard an increase in weight, which potentially allows for a continuation in progressive weight gain.

Additionally, the purpose of many of the aforementioned belt-like devices is simply to reduce the waist measurement temporarily, rather than to assist with managing a cycle of weight loss and weight gain.

The present Applicant has identified a potential advantage in providing a weight management device that provides an indication of over-eating in the form of a device that is fitted comfortably around a wearer's waist that becomes gradually tighter if the wearer over-eats, thus encouraging both acute and long term correction of eating habits.

Said device is therefore intended to not only produce a physical indication of weight gain by becoming gradually tighter if the wearer over-eats, but also promotes a psychological effect of making the wearer to become more aware of their dietary habits and accordingly, reduce regular occurrences of persistent over-eating that are likely to lead to progressive weight gain.

Accordingly, it is an objection of the present invention to provide a weight management device that alerts a wearer of weight gain as indicated by an increase in waist circumference and encourages a wearer to correct their eating and/or exercise habits to allow the device to fit comfortably again, thus reducing the likelihood of progressive weight gain.

Thus and in accordance with the present invention there is provided a weight management device for moderating bodyweight by providing an indication of changes in bodyweight, comprising a length of pliable material having a head end and a tail end and being substantially non-extensible longitudinally and having a non-releasable fastening means for engaging the head end and tail end wherein in use, said device is operable to be placed around a wearer's waist and become tighter in response to over-eating by the wearer, thus promoting corrective eating and/ or exercise habits.

It is further to be appreciated that a wearer may also experience an increase in waist measurement due to factors other than over-eating, including but not limited to changes in activity levels, and that accordingly, corresponding changes in fit of the device are intended to provide a relative indication of waist enlargement or reduction.

It is to be appreciated that "bodyweight" or "weight" as described herein are relative descriptions of a person's size based on waist circumference. Changes in waist circumference as highlighted by changes in fit of the device, may be indicative of a change in body shape and/or composition, such as for example, percentage body fat, and are not quantitative measurements of the wearer's actual bodyweight per se.

It will be appreciated that changes in waist circumference may provide a useful relative indication of changes in body fat in particular, which may not necessarily be reflected by a change in actual bodyweight. For example, it may be possible for a person to reduce their percentage body fat whilst increasing their proportion of lean muscle mass, which may not necessarily correspond to a decrease in actual bodyweight.

The weight management device most preferably comprises a length of material that may be pliable and thus capable of form-fitting around the waist of a wearer and preferably further comprises a fastening means, for securing said device around the waist. Said length of material may comprise a head end and a tail end.

Said material may comprise a cord-like construction and is most preferably of fine gauge so as to be comfortable and virtually undetectable to the wearer whilst in use, unless the wearer over-eats, in which case it will become more noticeable to the wearer, to encourage them to review their recent eating habits. Furthermore, due to the preferred discreet nature of the device, it may be substantially undetectable through clothing.

Said material may also comprise in certain embodiments, metals or other such suitable materials that may add aesthetic value to the device, whilst still permitting the device to be worn comfortably around the waist.

The weight management device is most preferably adapted to be fitted comfortably around the waist of a user. The tail end of the device may be engaged with the head end of said device and secured by means of a fastener, to retain said device around the waist. The device when in use, most preferably is flexible enough to move with the wearer's movement, so that it is as comfortable as possible during normal use and again, only becoming tighter in response to an increase in waist circumference, which may arise due to lifestyle changes.

The length of material comprising the weight management device preferably comprises soft and pliable materials to enable the device to be worn comfortably around a wearer's waist. Most preferably, the material will be of sufficient tensile strength to be resistant to snapping through normal use, however it is to be appreciated that the material is most preferably capable of breaking under strain, such as for example, if the device becomes accidently caught.

Further, the device may comprise hypo-allergenic materials and/ or fastenings, so as to minimise the risk of skin irritation whilst the device is in use. Additionally, it is preferred that the device comprises materials that are resistant to significant bacteria accumulation and also that are capable of withstanding daily hygiene tasks such as washing.

Preferably, the length of material comprising the weight management device may be secured around a wearer's waist by engaging a fastening means, which may be located at the head end of said material, with the tail end of said material, such that the length of material is joined at the fastening point so as to define a band-like arrangement.

The fastening means used to secure the device in position may be provided separately to the length of material that forms the band-like arrangement of the weight management device.

Most preferably, once a suitable length of material has been established around the waist, the fastener may be engaged and is then most preferably permanently secured, so as to prevent further re-adjustment of the device. This preferably discourages the wearer from continually expanding the circumference of the device to accommodate any additional weight gain, thus circumventing the principle of operation of the device, which is to promote a greater awareness of weight gain, to allow a person to make corrective changes to eating and/or exercise habits, before excessive weight gain is achieved. Any excess material may then be cut off, so as to promote a comfortable and unobtrusive fit of the device during normal use.

The weight management device is preferably operable to become tighter around the waist if a wearer overeats whilst the device is in use. This may make the wearer become conscious of the fact that they have overeaten and preferably encourages them to correct their eating and/or exercise habits acutely, so as to return their waist circumference to substantially the original measurement as compared to when said device was fitted, thus preferably enabling the device to return to its original comfortable fit.

Preferably, the weight management device provides a physical indication of changes in weight, by becoming tighter around the waist in response to over-eating by the wearer, such that they then become consciously aware of the fact that they have overeaten and accordingly, make necessary changes to their diet and/ or exercise regime to reduce their waist circumference again, such that said device regains its original fit around the waist.

The weight management device also preferably promotes a psychological effect of making the wearer to become more aware of their dietary habits by preventing voluntary expansion of the circumference of said device to accommodate any weight gain, by virtue of the non-adjustable fastener. Accordingly this heightened awareness encourages the wearer to reduce regular occurrences of persistent over-eating that are likely to lead to progressive weight gain and correct their eating and/ or exercise habits to return their waist to its original measurement and thus loosen the device.

Alternatively, the wearer can make a conscious decision to remove the device by purposefully removing it, such as for example by cutting it. It is anticipated that the physical act of having to cut the device off would act as a psychological barrier to removal of the device and encourage the wearer to consider the potential consequences of removing the device, such as for example, the likelihood of continuing weight gain.

Most preferably, the circumference of said band-like arrangement is determined by the wearer and is most optimally fitted so as to be comfortable and relatively loose-fitting. This enables the wearer to define a tolerance of weight gain/ waist enlargement that is permissible before the weight management device begins to tighten around the waist, thus enabling natural fluctuations in waist circumference that may arise during the day. It is preferred that the device only begins to feel tighter when the wearer has substantially overeaten, such that on noticing the increased tightness of the band, the wearer may choose to moderate their eating and/ or exercise habits temporarily until the device regain its original, comfortable fit.

Preferably, the fastening means may be opened so as to allow the band to be fitted, but once secured, is not re-adjustable, so as to prevent the device being made larger by the wearer, in an attempt to disguise or overcome any weight gain.

In an alternative embodiment however, it may be possible for the fastening means, once fitted and secured, to be adjusted so as to allow the device to be made smaller. This may assist wearers who have lost weight to continue to moderate their over-eating and continue a gradual and controlled weight loss, by providing a visual incentive and indication of progress as the band becomes gradually looser around the waist as more weight is lost.

The fastening means may comprise a ratchet-type mechanism or other form of adjustment mechanism that may allow controlled adjustment of the circumference of the device in use.

As the fastening arrangement most preferably prohibits enlargement of the device, the device may be removed by physically cutting the material comprising the belt-like portion of the device, which may encourage the wearer to more carefully consider the act of removing the device entirely, with respect to the potential for continued weight gain.

The invention will now be described further by way of example only and with reference to the drawings in which;
Figure 1 shows a view of the weight management device in an open configuration, prior to being fitted around the waist of a wearer and;
Figure 2 shows a view of the weight management device when the device has been securely fastened.

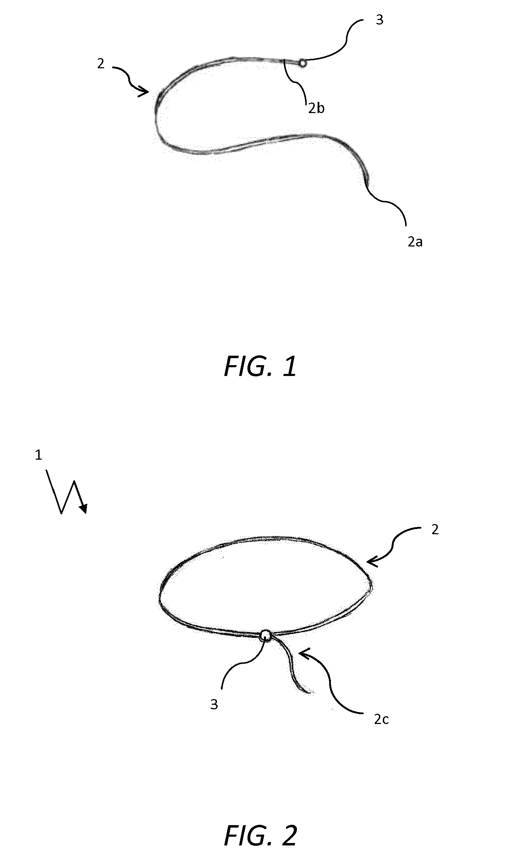

Referring now to Figure 1 there is shown a view of the weight management device 1 comprising a length of material 2 that forms a band-like arrangement when the device 1 is in use.

The material 2 comprises soft, pliable and non-elasticated fabrics, in order that longitudinal extension of the material is not permitted. This is to prevent the band of material 2 from being extended or enlarged to accommodate substantial weight gain when the device 1 is in use.

The material 2 comprises a tail end 2a and a head end 2b with said head end 2b being attachable to a fastening means 3. Said fastening means 3 is engageable with the tail end 2a of the material 2, so as to form a loop or band-like arrangement of material 2 as illustrated in Figure 2.

Referring now to Figure 2 there is shown a plan view of the weight management device 1 when the device has been securely fastened, as it would be around the waist of a wearer (not shown).

In use, the wearer (not shown) would take the weight management device 1 and place the unfastened length of material 2 around their waist. The tail end 2a of the device 1 would then be engaged with the fastening means 3, located substantially on the head end 2b in the present example, so as to adjustably connect the head end 2b of the material 2 with the tail end 2a, via the fastening means 3, so as to create a band-like arrangement around the waist.

The length and thus circumference of the band of material 2 is then adjusted by moving the tail end 2a in relation to the fastening means 3, so as to define a larger or smaller circumference as required to fit comfortably around the waist of a wearer.

It is anticipated that the most comfortable and appropriate fit will be one wherein the band of material 2 lies relatively loosely in adjacent communication with a wearer's waist, so as to allow for normal fluctuations in waist measurements that naturally occur throughout the day.

It is only when significant increases in waist measurement occur which may be suggestive of a significant increase in weight, that the band of material 2 will begin to feel tighter around the wearer's waist and thus promote a review of recent food intake and if necessary, an adjustment in subsequent food intake to reduce the waist measurement to its original size and thus restore the original, comfortable fit of the weight management device.

Once the appropriate fit has been established, the fastening means 3 may then be secured so as to prevent the device 1 from being re-adjusted. Any excess tail portion 2c of material 2 may then be cut off, so as to promote a comfortable and unobtrusive fit of the device 1 during normal use.

During normal use of the device 1, a wearer would eat as normal and, in the event that a healthy, balanced diet is maintained, the device 1 should remain substantially at its original fit, relative to the wearer's waist.

In the event that a wearer ate a significant quantity of food, or foods of high calorific value, it would be anticipated that there would be an acute increase in waist measurement which if maintained, would likely be indicative of an increase in weight. The band 2 of the device 1 would therefore feel tighter around the waist of the wearer, as a direct result of the increase in waist circumference.

The feeling of tightness is then detected by the wearer who then chooses to moderate the quantity and/ or type of food or increase the level of activity over the subsequent few days, which should result in the waist measurements returning to substantially the original size as compared to when the device 1 was fitted.

It is anticipated that over a period of time, the device 1 assists the wearer in becoming aware of their dietary habits, in particular portion size and food types, so that they become more able to regulate a healthy, balanced diet and thus manage their weight effectively.

It is to be appreciated that the invention as described herein is designed to assist persons who regularly or periodically over-eat, to prevent a continued process of weight gain. The device preferably serves as an indication of potential over eating which encourages the wearer to consider their recent dietary and exercise activity and if deemed appropriate, temporarily modify their eating and/ or exercise habits to cause a reduction in waist measurement, thus returning to an original weight.

The process of having to physically cut the device off to remove it (as opposed to being able to just enlarge it) in response to significant weight gain, provides a psychological indication of the potential consequences of removing the band which are the likelihood of increasing weight gain, unless eating habits are moderated.

The effect of using the weight management device as described herein is that, particularly for people who tend to have very pronounced fluctuations in weight, a continued cycle of weight loss and weight gain can be controlled, as the wearer is provided with an early indication of weight gain, thus enabling them to act more quickly to prevent further weight gain by moderating any excessive eating habits at an earlier stage than they might otherwise have done. This minimises extreme fluctuations in weight and promotes a greater awareness of healthy eating and weight maintenance.

It is of course to be appreciated that the invention is not to be restricted to the details of the embodiments described above which are described by way of example only.

## Claims

1. A weight management device for moderating bodyweight by providing an indication of changes in bodyweight, comprising a length of pliable material having a head end and a tail end and being substantially non-extensible longitudinally and having a non-releasable fastening means for engaging the head end and tail end wherein in use, said device is operable to be placed around a wearer's waist and become tighter in response to over-eating by the wearer, thus promoting corrective eating and/or exercise habits.

2. A weight management device in accordance with claim 1 wherein the pliable material comprises a cord-like material.

3. A weight management device in accordance with claim 1 or claim 2 wherein the pliable material comprises affine gauge cord-like material.

4. A weight management device in accordance with any one of the preceding claims wherein the pliable material comprises metals or other such aesthetically pleasing materials.

5. A weight management in accordance with any one of the preceding claims wherein device when in use is flexible enough to move with the wearer's movement.

6. A weight management device in accordance with any one of the preceding claims wherein the material will be of sufficient tensile strength to be resistant to snapping through normal use, however is capable of breaking under strain.

7. A weight management device in accordance with any one of the preceding claims comprising of hypo-allergenic materials and/or fastenings, so as to minimise the risk of skin irritation whilst the device is in use.

8. A weight management device in accordance with any one of the preceding claims wherein the device may be secured around a wearer's waist by engaging a fastening means, which may be located at the head end of said material, with the tail end of said material, such that the length of material is joined at the fastening point so as to define a band-like arrangement.

9. A weight management device in accordance with any one of the preceding claims wherein the fastening means used to secure the device in position may be provided separately to the length of material that forms the band-like arrangement of the weight management device.

10. A weight management device in accordance with claim 8 or claim 9 wherein the fastening means may comprise a ratchet-type mechanism or other form of adjustment.

11. A weight management device in accordance with any one of the preceding claims wherein once a suitable length of material has been established around the waist, the fastener may be engaged and is then most preferably permanently secured.

12. A weight management device in accordance with the preceding claim wherein the fastening means, once fitted and secured, may be adjusted so as to allow the device to be made smaller but not larger.

13. A weight management device substantially as hereinbefore described and with reference to the accompanying drawings.
